(19) Europäisches Patentamt
European Patent Office
Office européen des brevets

(11) **EP 3 659 703 A1**

(12) **EUROPEAN PATENT APPLICATION**

(43) Date of publication:
**03.06.2020 Bulletin 2020/23**

(51) Int Cl.:
*B01J 37/02* (2006.01)          *B01J 19/30* (2006.01)
*B01J 23/50* (2006.01)          *B01J 23/66* (2006.01)
*B01J 23/68* (2006.01)          *B01J 35/02* (2006.01)
*B01J 35/10* (2006.01)          *C07D 301/10* (2006.01)

(21) Application number: **18208856.7**

(22) Date of filing: **28.11.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BASF SE**
**67056 Ludwigshafen am Rhein (DE)**

(72) Inventor: **KARPOV, Andrey**
**67056 Ludwigshafen (DE)**

(74) Representative: **Reitstötter Kinzebach**
**Patentanwälte**
**Sternwartstrasse 4**
**81679 München (DE)**

(54) **CATALYST FOR PRODUCING ETHYLENE OXIDE BY GAS-PHASE OXIDATION**

(57) A shaped catalyst body for producing ethylene oxide by gas-phase oxidation of ethylene, comprising silver deposited on a porous refractory support, the shaped catalyst body having a first face side surface, a second face side surface and a circumferential surface, characterized by a content of at least 20 wt.-% of silver, relative to the total weight of the shaped catalyst body; a cylinder structure with n void spaces running in the cylinder periphery along the cylinder height to form an n-lobed structure, wherein n is 2, 3, 4, 5 or 6; n passageways extending from the first face side surface to the second face side surface, each passageway being assigned to one lobe, wherein neighboring passageways are arranged essentially equidistantly to each other; an n-fold rotational symmetry; a shortest distance A between two neighboring passageways in the range of 1.0 to 2.0 mm; a shortest distance B between each passageway and the circumferential surface in the range of 1.1 to 2.0 mm; and a BET surface area in the range of 1.6 to 3.0 m²/g. The shaped catalyst bodies allow for a favorable balance between mechanical stability, pressure drop and selectivity. The invention further relates to a reactor tube packed with the shaped catalyst bodies, a process for producing ethylene oxide by gas-phase oxidation of ethylene, comprising reacting ethylene and oxygen in the presence of the shaped catalyst bodies and to a process for preparing a shaped catalyst bodies.

## Fig. 1a

EP 3 659 703 A1

**Description**

The present invention relates to a shaped catalyst body for producing ethylene oxide, a process for producing ethylene oxide by gas-phase oxidation of ethylene and a process for preparing a shaped catalyst body.

Ethylene oxide is produced in large volumes and is primarily used as an intermediate in the production of several industrial chemicals. In the industrial oxidation of ethylene to ethylene oxide, heterogeneous catalysts comprising silver are used. To carry out the heterogeneously catalyzed gas-phase oxidation, a mixture of an oxygen-comprising gas, such as air or pure oxygen, and ethylene is generally passed through a plurality of tubes which are arranged in a reactor in which a packing of shaped catalyst bodies is present.

Catalyst performance is characterized by selectivity, activity, longevity of catalyst activity, and mechanical stability. Moreover, the performance in the reactor tubes is characterized by the packing density of the catalyst in the volume of the tubes and pressure drop across the catalyst bed. Selectivity is the molar fraction of the converted olefin yielding the desired olefin oxide. Even small improvements in selectivity and the maintenance of selectivity over longer time yield huge dividends in terms of process efficiency.

In an effort to optimize these various properties several approaches have been suggested, including variation of the geometrical shape of the catalyst bodies.

WO 2012/091898 A2 describes a catalyst useful for the epoxidation of olefins comprising a carrier with at least three lobes having a contoured shape and a plurality of passageways.

WO 2004/101144 A1 describes a catalyst useful in the epoxidation of ethylene in the shape of a hollow cylinder having a small inside (bore) diameter so as to improve the packing density.

US 2015/0119590 A1 describes silver catalysts suitable for producing ethylene oxide by oxidation of ethylene, comprising $\alpha$-alumina supports in the form of five-hole cylinder bodies.

US 4,837,194 describes catalysts for the production of alkylene oxides comprising an $\alpha$-alumina support in the form of cylinders pierced by seven longitudinal holes.

WO 2009/114411 A2 describes a geometrically shaped solid carrier for epoxidizing an olefin having thin walls of preferably less than 2.5 mm.

CN 102688784 A describes an alumina support for a silver catalyst suitable for olefin epoxidation, comprising a cylindrical substrate with a central passage, wherein the alumina support further comprises at least three partially cylindrical leaflets.

DE 10 2005 019 596 A describes a multilobed catalyst carrier with a plurality of smaller outer passageways arranged around a larger central passageway.

It is desirable for shaped catalyst bodies to have a high surface area so as to allow for a high rate of oxidation. The surface area of shaped catalyst bodies is, on the one hand, determined by the porosity of the shaped catalysts bodies. Pores are understood to be voids or interconnected channels accessible from the outside, having a diameter of 100 $\mu$m or less. The surface area of shaped catalyst bodies is also determined by the geometric surface area of the shaped catalyst body. The geometric surface area of a shaped catalyst body is defined by the external, macroscopic dimensions of the shaped catalyst body taking into account the cross-sectional area, the height and the number of internal passageways, if any. Internal passageways are macroscopic holes or conduits extending through the catalyst bodies, generally having diameters of 0.1 mm or more.

It has now been found that the selectivity strongly depends on catalyst shape. Without wishing to be bound by theory, it is believed that while ethylene usually passes into and out of the pores easily, the diffusion of the more bulky ethylene oxide takes significantly longer. Under the thus prolonged exposure, ethylene oxide may undergo undesired consecutive reactions induced by the catalyst, which reduces the overall selectivity of the process.

In order to allow for a quick diffusion of ethylene oxide, it would appear favorable to provide a shaped catalyst body wherein the pore volume is located predominantly close to the geometric surface of the shaped catalyst body, allowing ethylene oxide to pass out of the pores more quickly and avoiding long diffusion pathways within the catalyst body. One way of providing a shaped catalyst body with pores close to the geometric surface is to limit the size of the shaped catalyst bodies altogether, or to resort to flat particle shapes, or to utilize micronized catalyst particles (catalyst fines). However, limiting the size or thickness of the shaped catalyst bodies usually leads to an increase in the packing density and an increased resistance to the gas flowing through the packing. This causes an increase in the pressure drop which has to be overcome in order to force the reaction gas mixture through the catalyst packing.

Catalyst geometries with passageways extending through the solid catalyst have been suggested. In this case, a catalyst with pores close to the geometric surface may be provided by limiting the wall thickness between the passageways, or between the passageways and the circumferential surface of the catalyst particles. However, this approach is subject to limitations because excessively reducing the thickness of the walls of the catalyst particles generally also reduces their mechanical stability and crush strength. Catalyst bodies with lower crush strength and higher attrition tend to produce more broken catalyst bodies and more catalyst fines during handling, shipment, and installation into a commercial reactor. These broken catalyst bodies and catalyst fines create increased pressure drop in the commercial reactor tubes during normal operation and are thus unfavorable. Practically, geometries with extremely thin walls are useful only with catalysts of limited porosity, whose mechanical strength is increased due to their low porosity. Such lower porosity would however have a detrimental impact on the rate of the oxidation.

On the other hand, high mechanical strength may be achieved by reducing the size (diameter) of the passageways, and/or increasing the wall thickness. As described above, this approach has the drawbacks of increased mass transport limitations and increased pressure drop. Furthermore, passageways of small size may disadvantageously become clogged with silver complex solution during the manufacturing process of the shaped catalyst bodies.

It is an object of the present invention to provide a catalyst allowing for a balance between these factors.

The object is achieved by providing a shaped catalyst body for producing ethylene oxide by gas-phase oxidation of ethylene, comprising silver deposited on a porous refractory support, the shaped catalyst body having a first face side surface, a second face side surface and a circumferential surface, characterized by

- a content of at least 20 wt.-% of silver, relative to the total weight of the shaped catalyst body;
- a cylinder structure with n void spaces running in the cylinder periphery along the cylinder height to form an n-lobed structure, wherein n is 2, 3, 4, 5 or 6;
- n passageways extending from the first face side surface to the second face side surface, each passageway being assigned to one lobe, wherein neighboring passageways are arranged essentially equidistantly to each other;
- an n-fold rotational symmetry;
- a shortest distance A between two neighboring passageways in the range of 1.0 to 2.0 mm;
- a shortest distance B between each passageway and the circumferential surface in the range of 1.1 to 2.0 mm; and
- a BET surface area in the range of 1.6 to 3.0 $m^2/g$.

The shaped catalyst body is characterized by a cylinder structure which has n void spaces, e.g., grooves or furrows, running in the cylinder periphery along the cylinder height. The void spaces form an n-lobed structure, and n is 2, 3, 4, 5 or 6. Preferably, n is 3, 4 or 5, more preferably 3 or 4.

An n-lobed structure is understood to be a structure with n lobes. Thus, when n is 2, the structure is a bilobed structure; when n is 3, the structure is a trilobed structure; when n is 4, the n-lobed structure is a tetralobed structure; when n is 5, the n-lobed structure is a pentalobed structure; and when n is 6, the n-lobed structure is a hexalobed structure.

An axis parallel to the cylinder height and the first face side surface or the second face side surface, respectively, are essentially perpendicular but may deviate therefrom by an angle of up to 30°, such as up to 10° or 20°.

The shaped catalyst body is further defined by n passageways extending from the first face side surface to the second face side surface. As they display a large free surface area in their cross-section, the shaped bodies oppose a lower resistance to gas flow, with consequently lower pressure drops.

The n-lobed structure has a cross-section perpendicular to the cylinder height with the circumscribed circle diameter $d_{cc}$. "Circumscribed circle" means the smallest circle that completely contains the n-lobed cross-section within it. Preferably, the cross-section has a quotient of the diameter of the inscribed circle $d_{ic}$ over the diameter of the circumscribed circle $d_{cc}$ of at most 0.9, preferably in the range of 0.55 to 0.85 and most preferably in the range of 0.6 to 0.8. "Inscribed circle" means the largest possible circle that can be drawn inside the n-lobed cross-section. A quotient of $d_{ic}$ over $d_{cc}$ in this range allows for a lobe size which balances surface area and mechanical strength.

The circumscribed circle diameter $d_{cc}$ is usually in the range of 5 to 80 mm, preferably 5 to 20 mm and especially 5 to 10 mm, most preferably 6 to 10 mm. Generally, the quotient of the diameter $d_{cc}$ over the height h of the shaped body is in the range of 0.25 to 2.0, preferably 0.5 to 1.5 and more preferably 0.75 to 1.25, for example 0.80 to 1.20 or most preferably 0.90 to 1.15. A value of $d_{cc}/h$ in this range allows for especially favorable properties of the shaped catalyst body, in particular with regard to the pressure drop.

The height h of the shaped body (equaling the cylinder height) is understood to mean the distance between the two face sides along the central axis of the cylinder. The cylinder height is preferably in the range of 6 to 12 mm, more preferably in the range of 6 to 10 mm.

The passageways of the shaped catalyst body may have any technically feasible geometric form, such as an elliptical, polygonal or pincushion-shaped cross-section. An elliptical cross-section may be chosen among circular and oval shapes, for example. A polygonal cross-section may be chosen among rectangular and square shapes, for example. A pincushion-shaped cross-section is understood to comprise edges bowed inwards, i.e., to the center of the pincushion-shape. A preferred pincushion-shaped cross-section may have the shape of a square with all four edges bowed towards the center of the square.

The passageways are each assigned to one lobe, wherein neighboring passageways are arranged essentially equidistantly to each other. "Essentially equidistant" is understood to mean equidistant, save for the inevitable fluctuations and deviations inherent to the manufacturing process of the carrier. In general, essentially equidistant means a difference of no more than 10%, preferably no more than 5%, most preferably no more than 3% of the mean distance between neighboring passageways.

Preferably, the passageways are arranged symmetrically in the lobes. A passageway being arranged symmetrically in a lobe is understood to mean that a line running from the center of the circumscribed circle to the point of greatest extent of the lobe bisects the passageway into two mirror-symmetrical halves of the same area. Preferably, the line running from the center of the circumscribed circle to the point of greatest extent of the lobe also bisects the lobe into two mirror-symmetrical halves of the same area.

In one embodiment, the cross-section of the n-lobed structure has the shape of a circle with n indentations, each indentation having the shape of an elliptical segment. The indentations are of essentially identical size and arranged equidistantly to each other, yielding an n-lobed shape with an n-fold rotational symmetry. Shaped bodies having such a structure favorably roll well on an inclined surface, allowing for improved handling both in the production of the shaped catalyst bodies and in their use, for example when packing a reactor tube with the shaped catalyst bodies. Each passageway is assigned to a lobe and preferably, the passageway is arranged symmetrically in the lobe. Preferably, each indentation is mirror-symmetric with regard to a line running from the center of the circle through the middle of the indentation, which line does not touch any of the passageways.

In another embodiment, the cross-section of the n-lobed structure has the shape of a substantially equilateral, equiangular polygon having n sides, with elliptical segments attached to each side. In this case, the cross-section of the passageway assigned to a lobe lies partially within an elliptical segment and partially within the polygon. Preferably, each elliptical segment is mirror-symmetric with regard to a line running from the center of the rectangle through the middle of the rectangle side to which the elliptical segment is attached, which line bisects one passageway. It is understood that the passageway is bisected into two halves of the same area, preferably the same area and shape. In a preferred embodiment, the elliptical segments are circular segments, preferably of essentially identical size. The elliptical segments preferably traverse the side they are attached to and meet at the corners of the polygon. This yields an n-lobed shape having individual lobes and indwelling, apical, interlobular interstices.

The shaped catalyst body has an n-fold rotational symmetry. Such an arrangement of passageways allows for an essentially uniform wall thickness, especially when the passageways are located symmetrically in each of the lobes.

Preferably, the cross-sectional area of each of the passageways is independently in the range of 0.5 to 13.0 $mm^2$, preferably in the range of 1.0 to 3.2 $mm^2$, most preferably in the range of 1.2 to 3.0 $mm^2$. In especially preferred embodiments, the cross-sectional area of each of the passageways is independently in the range of 1.3 to 2.8 $mm^2$, preferably in the range of 1.4 to 2.7 $mm^2$, most preferably 1.5 to 2.6 $mm^2$. It was found that such an arrangement allows for high mechanical stability while limiting wall thickness. Preferably, the cross-sectional area is essentially identical for each passageway.

In a preferred embodiment, the passageways have an elliptical cross-section, especially a circular cross-section.

In a preferred embodiment, the shaped catalyst body has a trilobe structure with three passageways extending from the first face side surface to the second face side surface. In this embodiment, n is 3 and the shaped catalyst body has a 3-fold rotational symmetry. The shape of the passageways may deviate from the 3-fold rotational symmetry. Preferably, however, the shape of the passageways is part of the 3-fold rotational symmetry. Thus, it is preferable that the three passageways have essentially identical, symmetrical cross-sections, and that they are arranged so that their center points form an equilateral triangle. Most preferably, each of the three passageways is arranged symmetrically within one of the three lobes.

In another preferred embodiment, the shaped catalyst body has a tetralobe structure with four passageways extending from the first face side surface to the second face side surface. In this embodiment, n is 4 and the shaped catalyst body has a 4-fold rotational symmetry. The shape of the passageways may deviate from the 4-fold rotational symmetry. Preferably, however, the shape of the passageways is part of the 4-fold rotational symmetry. Thus, it is preferable that the passageways have essentially identical, symmetrical cross-sections, and that they are arranged so that their center points form a square. Most preferably, each of the four passageways is arranged symmetrically within one of the four lobes.

In another preferred embodiment, the shaped catalyst body has a pentalobe structure with five passageways extending from the first face side surface to the second face side surface. In this embodiment, n is 5 and the shaped catalyst body has a 5-fold rotational symmetry. The shape of the passageways may deviate from the 5-fold rotational symmetry. Preferably, however, the shape of the passageways is part of the 5-fold rotational symmetry. Thus, it is preferable that the passageways have essentially identical, symmetrical cross-sections, and that they are arranged so that their center points form an equilateral, equiangular pentagon. Most preferably, each of the five passageways is arranged symmetrically within one of the five lobes.

The shortest distance A between two neighboring passageways is in the range of 1.0 to 2.0 mm, preferably 1.1 to 1.9 mm, more preferably 1.2 to 1.8 mm. The shortest distance B between each passageway and the circumferential surface is in the range of 1.1 to 2.0 mm, preferably 1.2 to 1.9 mm, more preferably 1.25 to 1.8 mm. A minimum wall thickness is provided by the definition of the shortest distances A and B, allowing for a high stability of the shaped catalyst body. Further, a maximum wall thickness is provided so as to avoid long diffusion pathways.

Preferably, the ratio of the shortest distance A to the shortest distance B is in the range of 0.6 to 1.3, preferably 0.7 to 1.2, most preferably 0.7 to 1.0. A ratio of the shortest distance A to the shortest distance B in this range allows for an essentially uniform wall thickness.

The shape of the inventive catalyst bodies allows for extending the geometric volume / geometric surface ($SA_{geo}/V_{geo}$) ratios beyond those available in simple cylindrical or spherical forms. The geometric surface area $SA_{geo}$ and the geometric volume $V_{geo}$ is derived from the external, macroscopic dimensions of the shaped catalyst body taking into account the cross-sectional area, the height and the number of internal passageways. In other words, the geometric volume $V_{geo}$ of the catalyst bodies is the volume of a solid n-lobed structure having the same outer dimensions, minus the volume occupied by the passageways.

Likewise, the geometric surface area $SA_{geo}$ is made up of the circumferential surface, the first and second face side surface and the surface defining the passageways. The first and second face side surface, respectively, is the surface area enclosed by the circumferential line of the face side, minus the cross-sectional areas of the passageways. The surface defining the passageways is the surface area lining the passageways.

Preferably, the quotient of the geometric surface of the shaped catalyst body $SA_{geo}$ over the geometric volume of the shaped catalyst body $V_{geo}$ is at least 1.1 $mm^{-1}$ and at most 10 $mm^{-1}$. Preferably, the quotient of $SA_{geo}$ over $V_{geo}$ is in the range of 1.15 $mm^{-1}$ to 5.0 $mm^{-1}$, more preferably in the range of 1.2 $mm^{-1}$ to 2.0 $mm^{-1}$, most preferably in the range of 1.3 to 1.45 $mm^{-1}$. A quotient of $SA_{geo}$ over $V_{geo}$ in this range makes it possible for a better contact of the reaction gases with the catalyst surface to be obtained, which favors the conversion of the reactants and limits the inner diffusion phenomena, with a resulting increase in reaction selectivity.

Preferably, the ratio of the total cross-sectional area of the passageways to the cross-sectional area of the shaped catalyst body is in the range of 0.1 to 0.4, more preferably in the range of 0.1 to 0.3. The total cross-sectional area of the passageways is understood to mean the sum of the cross-sectional areas of the passageways. The cross-sectional area of the shaped catalyst body is understood to mean the surface area enclosed by the circumferential line of the face side. The cross-sectional area of the shaped catalyst body includes the cross-sectional areas of the passageways. Accordingly, the ratio of the total cross-sectional area of the passageways to the cross-sectional area of the shaped catalyst body reflects the share of the passageway area of the cross-sectional area of the shaped catalyst body.

The shaped catalyst body of the invention has a high mechanical stability. Preferably, the shaped catalyst body has a side crush strength of at least 50 N. The term "side crush strength" refers to the force necessary to break the shaped catalyst body at its weakest pressure point. For the purposes of the present invention, the side crush strength is the force which fractures the shaped catalyst body located between two flat parallel plates, with the height of the cylindrical body parallel to the flat parallel plates.

Shaped catalyst supports are generally obtained by extrusion of a suitable precursor material through an extrusion apparatus, and subsequent drying and calcination of the extrudate. The extrudate is preferably a porous refractory support, on which silver is subsequently deposited, e.g. by use of a silver impregnation solution. Further to the shaped catalyst body having a high side crush strength, it is also favorable for the still moist freshly extruded body to have a high side crush strength. The shape of the inventive catalyst bodies favorably allows for a high crush strength of the freshly extruded body. Alternatively, the shaped catalyst supports can be obtained by tableting of a suitable precursor material.

The BET surface area and the porous nature of the catalyst as determined by mercury porosimetry contribute substantially to the active surface area at which the catalytic reaction takes place. For the internal surfaces of the catalyst body to be utilized effectively, the feed gases must diffuse through the pores to reach the internal surfaces, and the reaction products must diffuse away from those surfaces and out of the catalyst body. The catalytic performance is influenced by the catalyst's pore structure.

The shaped catalyst body is characterized by a BET surface area in the range of 1.6 to 3.0 $m^2/g$, preferably 1.8 to 2.8 $m^2/g$, more preferably 2.0 to 2.6 $m^2/g$, most preferably 2.3 to 2.5 $m^2/g$. The BET method is a standard, well-known method and widely used method in surface science for the measurements of surface areas of solids by physical adsorption of gas molecules. The BET surface is determined according to DIN ISO 9277 herein, unless stated otherwise.

The porosity of the shaped catalyst body may further be characterized by its Hg pore volume. Preferably, the shaped catalyst body has a total Hg pore volume of 0.2 mL/g to 0.6 mL/g, preferably 0.25 mL/g to 0.5 mL/g, especially preferably 0.3 mL/g to 0.4 mL/g, as determined by mercury porosimetry. Mercury porosimetry may be performed using a Micrometrics AutoPore IV 9500 mercury porosimeter (140 degrees contact angle, 485 dynes/cm Hg surface tension, 60000 psia max head pressure). The Hg porosity is determined according to DIN 66133 herein, unless stated otherwise. It is believed that a Hg pore volume in this range allows for a favorable duration of exposure of the obtained ethylene oxide to the catalyst.

Preferably, the shaped catalyst body has a multimodal pore size distribution with at least one pore size distribution maximum in the range of 0.1 to 3.0 $\mu$m. Preferably at least 40% of the total Hg pore volume of the shaped catalyst body stems from pores with a diameter in the range of 0.1 to 3.0 $\mu$m. More preferably 50% to 90%, in particular 50% to 80% of the total pore volume of the shaped catalyst body stems from pores with a diameter in the range of 0.1 to 3.0 $\mu$m. Preferably, the shaped catalyst body further has at least one pore size distribution maximum in the range of 8.0 to 100 µm. Preferably at most 50% of the total Hg pore volume of the shaped catalyst body stems from pores with a diameter of 8.0 to 100 µm. More preferably 10% to 50%, in particular 20% to 40% of the total Hg pore volume of the shaped catalyst body stems from pores with a diameter of 8.0 to 100 µm. It is believed that such a multimodal pore size distribution is advantageous because it allows to achieve high catalyst selectivity, activity and durability.

The shaped catalyst body comprises silver deposited on a porous refractory support, wherein the content of silver is at least 20 wt.-%, relative to the total weight of the shaped catalyst body. Preferably the shaped catalyst body has a content of at least 22 wt.-%, more preferably at least 23 wt.-% most preferably at least 25 wt.-%, relative to the total weight of shaped catalyst body.

For example, the catalyst may comprise 20 to 45 % wt.-% of silver, relative to the total weight of the shaped catalyst body. A preferred catalyst comprises 22 to 35 wt.-% of silver, more preferably 23 to 30 wt.-% of silver, e.g., 25 to 30 wt.-% relative to the total weight of the shaped catalyst body. A silver content in this range allows for a favorable balance between turnover induced by each shaped catalyst body and cost-efficiency of preparing the shaped catalyst body.

The bulk density of a catalyst is typically much higher than the packed density in, e.g., a tubular reactor such as commercially used for the production of ethylene oxide. The packed density of the shaped catalyst bodies is preferably at least 700 g/L, more preferably 750 to 1200 g/L, in particular 800 to 1000 g/L, as measured in a reactor tube with an inner diameter of 39 mm. The silver density per liter of a tubular reactor packed with catalyst in accordance with this invention is often at least 140 g/L, e.g., at least 180 g/L, preferably at least 200 g/L, in particular at least 220 g/L as measured in a reactor tube with an inner diameter of 39 mm. The silver density per liter of a tubular reactor can be calculated by multiplying packed catalyst density by the silver content of the catalyst bodies.

Alternatively, the packed density of the shaped catalyst bodies is preferably at least 600 g/L, more preferably 700 to 1100 g/L, in particular 750 to 1000 g/L, as measured in a reactor tube with an inner diameter of 26 mm. The silver density per liter of a tubular reactor packed with catalyst in accordance with this invention is often at least 120 g/L, e.g., at least 165 g/L, preferably at least 185 g/L, in particular at least 205 g/L as measured in a reactor tube with an inner diameter of 26 mm.

Besides silver, the shaped catalyst body may comprise one or more promoting species. A promoting species denotes a component that provides an improvement in one or more of the catalytic properties of the catalyst when compared to a catalyst not containing said component. The promoting species can be any of those species known in the art that function to improve the catalytic properties of the silver catalyst. Examples of catalytic properties include operability (resistance to runaway), selectivity, activity, turnover and catalyst longevity.

The shaped catalyst body may comprise a promoting amount of a transition metal or a mixture of two or more transition metals. Suitable transition metals can include, for example, the elements from Groups IIIB (scandium group), IVB (titanium group), VB (vanadium group), VIB (chromium group), VIIB (manganese group), VIIIB (iron, cobalt, nickel groups), IB (copper group), and IIB (zinc group) of the Periodic Table of the Elements, as well as combinations thereof. More typically, the transition metal is an early transition metal, i.e., from Groups IIIB, IVB, VB or VIB, such as, for example, hafnium, yttrium, molybdenum, tungsten, rhenium, chromium, titanium, zirconium, vanadium, tantalum, niobium, or a combination thereof. In one embodiment, the transition metal promoter(s) is (are) present in a total amount from 150 ppm to 5000 ppm, typically 225 ppm to 4000 ppm, most typically from 300 ppm to 3000 ppm, expressed in terms of metal(s) relative to the total weight of the shaped catalyst body.

Of the transition metal promoters listed, rhenium (Re) is a particularly efficacious promoter for ethylene epoxidation high selectivity catalysts. The rhenium component in the shaped catalyst body can be in any suitable form, but is more typically one or more rhenium- containing compounds (e.g., a rhenium oxide) or complexes.

Preferably, the shaped catalyst body comprises 400 to 2000 ppm by weight of rhenium, relative to the total weight of the shaped catalyst body. It is preferred that the shaped catalyst body comprises 560 to 1700 ppm by weight of rhenium, more preferably 610 to 1500 ppm by weight of rhenium, most preferably 700 to 1400 ppm by weight of rhenium, e.g., 740 to 1300 ppm by weight of rhenium, relative to the total weight of the shaped catalyst body.

In some embodiments, the shaped catalyst body may include a promoting amount of an alkali metal or a mixture of two or more alkali metals. Suitable alkali metal promoters include, for example, lithium, sodium, potassium, rubidium, cesium or combinations thereof. The amount of alkali metal, e.g. potassium, will typically range from 50 ppm to 5000 ppm, more typically from 300 ppm to 2500 ppm, most typically from 500 ppm to 1500 ppm expressed in terms of the alkali metal relative to the total weight of the shaped catalyst body. The amount of alkali metal is determined by the amount of alkali metal contributed by the porous refractory support and the amount of alkali metal contributed by the impregnation solution described below.

Combinations of heavy alkali metals like cesium (Cs) or rubidium (Rb) with light alkali metals like lithium (Li), sodium (Na) and potassium (K) are particularly preferred.

Cesium is an especially preferred alkali metal promotor. Preferably, the shaped catalyst body comprises 260 to 1750 ppm by weight of cesium, relative to the total weight of the shaped catalyst body. It is preferred that the shaped catalyst body comprises 400 to 1500 ppm by weight of cesium, more preferably 530 to 1500 ppm by weight of cesium, most preferably 600 to 1330 ppm by weight of cesium, e.g., 730 to 1330 ppm by weight of cesium, relative to the total weight of the shaped catalyst body.

Preferably the shaped catalyst body contains at least two light alkali metals, selected from sodium, potassium and lithium. Most preferably the shaped catalyst body contains sodium, potassium and lithium.

Preferably, the shaped catalyst body comprises 40 to 1170 ppm by weight of potassium, relative to the total weight of the shaped catalyst body. It is preferred that the shaped catalyst body comprises 100 to 400 ppm by weight of potassium, most preferably 140 to 250 ppm by weight of potassium. The amount of potassium is determined by the amount of potassium contributed by the porous refractory support and the amount of potassium contributed by the impregnation solution described below.

Preferably, the shaped catalyst body comprises 100 to 2000 ppm by weight of lithium, relative to the total weight of the shaped catalyst body. It is preferred that the shaped catalyst body comprises 150 to 1500 ppm by weight of lithium, most preferably 300 to 750 ppm by weight of lithium. The amount of lithium is determined by the amount of lithium contributed by the porous refractory support and the amount of lithium contributed by the impregnation solution described below.

Preferably, the shaped catalyst body comprises 10 to 1000 ppm by weight of sodium, relative to the total weight of the shaped catalyst body. It is preferred that the shaped catalyst body comprises 20 to 500 ppm by weight of sodium, most preferably 30 to 250 ppm by weight of sodium. The amount of sodium is determined by the amount of sodium contributed by the porous refractory support and the amount of sodium contributed by the impregnation solution described below.

The shaped catalyst body may also include a Group IIA alkaline earth metal or a mixture of two or more Group IIA alkaline earth metals. Suitable alkaline earth metal promoters include, for example, beryllium, magnesium, calcium, strontium, and barium or combinations thereof. The amounts of alkaline earth metal promoters can be used in amounts similar to those used for the alkali or transition metal promoters.

The shaped catalyst body may also include a promoting amount of a main group element or a mixture of two or more main group elements. Suitable main group elements include any of the elements in Groups IIIA (boron group) to VIIA (halogen group) of the Periodic Table of the Elements. For example, the shaped catalyst body can include a promoting amount of sulfur, phosphorus, boron, halogen (e.g., fluorine), gallium, or a combination thereof.

The shaped catalyst body may also include a promoting amount of a rare earth metal or a mixture of two or more rare earth metals. The rare earth metals include any of the elements having an atomic number of 57-103. Some examples of these elements include lanthanum (La), cerium (Ce), and samarium (Sm). The amount of rare earth metal promoters can be used in amounts similar to those used for the transition metal promoters.

The shaped catalyst body comprises a porous refractory support. Any refractory support known in the art can be used. Suitable examples of refractory supports are described in EP 0902726, EP 1478458, EP 1675678, EP 3254756, WO 2004/101144, WO 2007/021472, WO 2008/054654, WO 2009/029414, WO 2010/008920, WO 2011/153390A1, WO 2012/143557A1, WO 2012/143559A1, WO 2013/077839A1 or US 2018/0021755.

Preferably, the refractory support is an aluminium oxide support. The aluminium oxide support usually comprises a high proportion of $\alpha$-$Al_2O_3$, for example at least 90 percent by weight, preferably at least 95 percent by weight, most preferably at least 97,5 percent by weight, based on the total weight of the support.

When packing a tubular reactor with the shaped catalyst bodies, it may be advantageous to choose the dimensions of the shaped catalyst bodies in consideration of the dimensions of the reactor tubes of the tubular reactor. Preferably, the ratio of the diameter of the circumscribed circle $d_{cc}$ of the shaped catalyst bodies to the inner diameter of a reactor tube is in the range of 0.15 to 0.35, more preferably 0.2 to 0.3.

Accordingly, there is further provided a reactor tube packed with a plurality of shaped catalyst bodies as described herein, wherein the ratio of the diameter of the circumscribed circle $d_{cc}$ of the shaped catalyst bodies to the inner diameter of the reactor tube is in the range of 0.15 to 0.35, preferably 0.2 to 0.3.

For example, it is advantageous to pack a reactor tube with an inner diameter of 39 mm with shaped catalyst bodies wherein the diameter of the circumscribed circle $d_{cc}$ is in the range of 8.5 to 9.0 mm, e.g., 8.75 mm. In the case of a reactor tube with an inner diameter of 26 mm, it is preferable to pack the reactor tube with shaped catalyst bodies wherein the diameter of the circumscribed circle $d_{cc}$ is in the range of 6.7 to 7.3 mm, e.g., 7.1 mm.

Further provided is a process for preparing a shaped catalyst body as defined in any one of the preceding claims, comprising

i) impregnating a refractory support having a BET surface area in the range of 1.4 to 2.5 $m^2$/g with a silver impregnation solution, preferably under reduced pressure; and optionally subjecting the impregnated refractory support to drying; and

ii) subjecting the impregnated refractory support to a calcination process;
wherein steps i) and ii) are optionally repeated.

In order to obtain a shaped catalyst body having high silver contents, steps i) and ii) can be repeated several times. In that case it is understood that the intermediate product obtained after the first (or subsequent up to the last but one) impregnation/calcination cycle comprises a part of the total amount of target Ag and / or promoter concentrations. The intermediate product is then again impregnated with the silver impregnation solution and calcined to yield the target Ag and / or promoter concentrations.

The aluminium oxide support may comprise impurities, such as sodium, potassium, iron, silica, magnesium, calcium, zirconium in an amount of 100 to 10000 ppm, based on the total weight of the support.

Any known impregnation processes known in the art can be used. Examples of suitable impregnation processes are described, e.g., in WO2013/066557 or WO2018/044992. Preferably, impregnation is conducted at a pressure of less than 250 mbar, more preferably at a pressure of less than 100 mbar.

Any calcination processes known in the art can be used. Suitable examples of calcination processes are described in US 5,504,052, US 5,646,087, US 7,553,795, US 8,378,129, US 8,546,297, US2014/0187417, EP 1893331 or WO2012/140614.

It is understood that all embodiments of the shaped catalyst body also apply to the process for preparing the shaped catalyst body, where applicable. For example, the refractory support of step i) is preferably characterized and obtainable by the embodiments described above.

The porous refractory support may be characterized by its BET surface area, total pore volume and pore size distribution, which are measured by mercury intrusion, and by its water absorption. It is assumed that certain ranges of BET surface area, total pore volume and pore size distribution of the support and of the water absorption of the support are preferred in order to provide an overall improved catalyst which provides high selectivity but still has a high side crush strength.

The BET surface area of the porous refractory support is preferably in the range of 1.4 to 2.5 $m^2/g$, more preferably 1.7 to 2.3 $m^2/g$, most preferably at least 1.8 $m^2/g$, e.g., 1.9 to 2.2 $m^2/g$. The BET surface is determined according to DIN ISO 9277 herein, unless stated otherwise.

The BET surface of the shaped catalyst body is usually higher in comparison to the BET surface area of the porous refractory support. The BET surface area of the shaped catalyst body is in the range of 1.6 to 3.0 $m^2/gm$, preferably 1.8 to 2.8 $m^2/g$, more preferably 2.0 to 2.6 $m^2/g$, most preferably at least 2.3 $m^2/g$, e.g., 2.3 to 2.5 $m^2/g$.

The BET surface area of the porous refractory support can be adjusted, for example, by combining two $\alpha$-$Al_2O_3$ particles of different sizes when the refractory support is prepared (see, e.g., EP 0 902 726 B1 or WO 2011/153390 A2) or varying the calcination temperature. As pointed out in WO 2012/143559 A1, an increase in the porosity can be achieved using organic additives having a placeholder function, thereby obtaining size distributions which are directly related to the grain sizes of the raw materials used. Additional methods for preparing support materials are described in WO 2012/143559 A1 and WO 2012/143557 A1.

The water absorption of the refractory support is an indicator for the suitability of the refractory support to have an active catalyst component, such as silver, deposited thereon, for example by impregnation with a silver impregnation solution. Excessively low water absorption indicates that the generally aqueous impregnation solution does not penetrate the pores of the refractory support to a desirable degree, and impregnation may not be sufficient to obtain a desirable amount of active catalyst component in the shaped catalyst body. Excessively high water absorption, on the other hand, may cause poor mechanical strength. In general, water absorption values correlate well with the total pore volume of the support determined by mercury intrusion. However, catalyst shaped bodies with small passageways, e.g., with passageways below 1.5 mm, may display significant deviations between water absorption and the total pore volume of the support, as a part of water might be retained by passageways.

When the water absorption is more than 5% higher than the total pore volume determined by mercury intrusion, the support is impregnated based on the total pore volume determined by mercury. In some embodiments it is preferable to impregnate only a part of the support based either on water absorption or on a total pore volume determined by mercury intrusion.

The water absorption of the refractory support can, for example, be in the range of 0.35 to 0.70 mL/g (mL of water/gram of support), preferably 0.38 to 0.65 mL/g, most preferably 0.41 to 0.60 mL/g. Water absorption refers to vacuum cold water uptake measured at a vacuum of 80 mbar. Vacuum cold water uptake is determined by placing about 100 g of support ("initial support weight") in a rotating flask, covering the support with deionized water, and rotating the rotary evaporator for 5 min at about 30 rpm. Subsequently, a vacuum of 80 mbar is applied for 3 min, the water and the support are transferred into a glass funnel, and the support is kept in the funnel for about 5 min with occasional shaking in order to ensure that adhering water runs down the funnel. The support is weighed ("final support weight"). The water absorption is calculated by subtracting the initial support weight from the final support weight and then dividing this difference by the initial support weight.

The ratio of water absorption of the support (mL of water/gram of support) to BET surface area of the support ($m^2$ of support/gram of support) is in the range of 0.18 to 0.33 mL/$m^2$, preferably in the range from 0.20 to 0.30 mL/$m^2$.

The refractory support may generally be obtained by extrusion of a suitable precursor material through a die, and subsequent drying and calcination of the extrudate. The cross-section of the die opening is adapted according to the above embodiments.

The extrusion die may comprise a matrix and mandrels, wherein the matrix essentially determines the form, size and position of the void spaces running in the cylinder periphery and the mandrels essentially determine the form, size and position of the passageways.

The geometry of the shape of the shaped catalyst body of the invention is defined by the ideal geometry of the extrusion apparatus through which the catalyst precursor mold is extruded. Generally, the geometry of the shape of the extrudate differs slightly from the ideal geometry of the extrusion apparatus, while essentially having the geometric properties described above. Absolute sizes of the shape are in general slightly lower than the sizes of the extrudate, due to high temperatures required to form alpha alumina and shrinkage upon cooling of the extrudate. The extent of the shrinkage depends on the applied calcination temperatures. Therefore, the size of the extrusion dies should be routinely adjusted in a way to account for the extrudate shrinkage during the subsequent calcination step.

Generally, the axes of the passageways run parallel. However, the shaped catalyst bodies may be slightly bent or twisted along its z axis (height). The shape of the cross-section of the passageways may be slightly different from the perfect geometrical shapes described above. When a large amount of shaped bodies is obtained, single passageways of a small number of the shaped catalyst bodies may be closed. Usually the face sides of the shaped catalyst bodies in the xy plane are more or less uneven, rather than smooth, due to the production process. The height of the shaped bodies (length of the shaped bodies in the z direction) is usually not exactly the same for all of the shaped catalyst bodies, but rather constitutes a distribution with an average height as its arithmetic mean.

The extrudate is cut into the desired length while still wet. Preferably, the extrudate is cut at an angle essentially perpendicular to its circumferential surface. In order to reduce undesirable deviations from the ideal geometry of the extrusion apparatus, the extrudate may alternatively be cut at a slanted angle of up to 30°, such as 10° or 20°, with regard to the angle perpendicular to the circumferential surface of the extrudate.

Aberrations from the ideal geometry as incurred in the extrusion process and/or the further processing of the extrudate, e.g. a cutting step, may generally also be present in the shaped catalyst body of the invention without essentially lessening the favorable effects of its geometry. The skilled person understands that perfect geometrical forms are fundamentally unobtainable due to the imprecision which is inherent to all production processes to some degree.

Any silver impregnation solution suitable for impregnating a refractory support known in the art can be used. Silver impregnation solutions typically contain silver oxalate, or a combination of silver oxide and oxalic acid, together in ethylenediamine. Suitable impregnation solutions are described in EP 0 716 884 A2, EP 1 115 486 A1, EP 1 613 428 A1, US 4,731,350, WO 2004/094055 A2, WO 2009/029419 A1, WO 2015/095508 A1, US 4,356,312, US 5,187,140, US 4,908,343, US 5,504,053 and WO 2014/105770 A1.

Any impregnation processes known in the art can be used. A suitable example of an impregnation process is described in WO 2013/066557.

It is possible to establish the desired composition of the catalyst with relative amounts of all metals as desired in only one impregnation. Alternatively, impregnation step i) may comprise multiple alternating impregnation and drying steps. The impregnation solution in a first impregnation step comprises for example silver and promotor, e.g. rhenium and/or cesium. Additional promotors, such as tungsten, lithium, and/or sulfur can, for example, be comprised by a silver impregnation solution used in a second or later impregnation step.

The impregnated refractory support is subjected to a calcination process ii). Any calcination processes known in the art can be used. Suitable examples of calcination processes are described in US 5,504,052, US 5,646,087, US 7,553,795, US 8,378,129, US 8,546,297, US 2014/0187417, EP 1893331 or WO 2012/140614. Calcination can be carried out in a pass-through mode or with at least partial recycling of the calcination gas.

The calcination process is usually carried out in a furnace. The type of furnace is not especially limited. For example, stationary circulating air furnaces, revolving cylindrical furnaces or conveyor furnaces may be used. The duration of the calcination process is generally in the range of 5 min to 20 h, preferably 5 min to 30 min. The temperature of the calcination process is generally in the range of 200 to 800 °C.

It may be favorable to sort the shaped catalyst body or its precursors according to size and use only a fraction of suitable size. This sorting may be performed after the calcination or at another suitable point of time during the production process. The sorting may be achieved by using suitable sieves. Shaped bodies which are larger or smaller than the desired dimensions may be recycled as return material to suitable points of the process. It may be preferable to subject the return material to further processing steps, such as a grinding step, prior to recycling.

Further provided is a process for producing ethylene oxide by gas-phase oxidation of ethylene, comprising reacting ethylene and oxygen in the presence of a shaped catalyst body as discussed above. It is understood that all embodiments of the shaped catalyst body also apply to the process for producing ethylene oxide in the presence of the shaped catalyst body, where applicable.

According to the invention, the epoxidation can be carried out by all processes known to those skilled in the art. It is possible to use all reactors which can be used in the ethylene oxide production processes of the prior art; for example externally cooled shell-and-tube reactors (cf. Ullmann's Encyclopedia of Industrial Chemistry, 5th edition, vol. A-10, pp. 117-135, 123-125, VCH-Verlagsgesellschaft, Weinheim 1987) or reactors having a loose catalyst bed and cooling tubes, for example the reactors described in DE-A 3414717, EP 0082609 and EP-A 0339748.

The epoxidation is preferably carried out in at least one tube reactor, preferably in a shell-and-tube reactor. On a commercial scale, ethylene epoxidation is preferably carried out in a multi-tube reactor that contains several thousand tubes. The catalyst is filled into the tubes, which are placed in a shell that is filled with a coolant. In commercial applications, the internal tube diameter is typically in the range of 20 to 40 mm (see, e.g., US 4,921,681) or more than 40 mm (see, e.g., WO2006/102189).

To prepare ethylene oxide from ethylene and oxygen, it is possible to carry out the reaction under conventional reaction conditions as described, for example, in DE-A 2521906, EP-A 0 014 457, DE-A 2300512, EP-A 0 172 565, DE-A 2454972, EP-A 0 357 293, EP-A 0 266 015, EP-A 0 085 237, EP-A 0 082 609 and EP-A 0 339 748. Inert gases such as nitrogen or gases which are inert under the reaction conditions, e.g. steam, methane, and also optionally reaction moderators, for example halogenated hydrocarbons such as ethyl chloride, vinyl chloride or 1,2-dichloroethane can additionally be mixed into the reaction gas comprising ethylene and molecular oxygen.

The oxygen content of the reaction gas is advantageously in a range in which no explosive gas mixtures are present. A suitable composition of the reaction gas for preparing ethylene oxide can, for example, comprise an amount of ethylene in the range from 10 to 80% by volume, preferably from 20 to 60% by volume, more preferably from 25 to 50% by volume and particularly preferably in the range from 25 to 40% by volume, based on the total volume of the reaction gas. The oxygen content of the reaction gas is advantageously in the range of not more than 10% by volume, preferably not more than 9% by volume, more preferably not more than 8% by volume and very particularly preferably not more than 7.5% by volume, based on the total volume of the reaction gas.

The reaction gas preferably comprises a chlorine-comprising reaction moderator such as ethyl chloride, vinyl chloride or 1,2-dichloroethane in an amount of from 0 to 15 ppm by weight, preferably in an amount of from 0.1 to 8 ppm by weight, based on the total weight of the reaction gas. The remainder of the reaction gas generally comprises hydrocarbons such as methane and also inert gases such as nitrogen. In addition, other materials such as steam, carbon dioxide or noble gases can also be comprised in the reaction gas.

The concentration of carbon dioxide in the feed (i.e. the gas mixture fed to the reactor) typically depends on the catalyst selectivity and the efficiency of the carbon dioxide removal equipment. Carbon dioxide concentration in the feed is preferably at most 3 vol-%, more preferably less than 2 vol-%, most preferably less than 1 vol-%, relative to the total volume of the feed. An example of carbon dioxide removal equipment is provided in US 6,452,027.

The above-described constituents of the reaction mixture may optionally each have small amounts of impurities. Ethylene can, for example, be used in any degree of purity suitable for the gas-phase oxidation according to the invention. Suitable degrees of purity include, but are not limited to, "polymer-grade" ethylene, which typically has a purity of at least 99%, and "chemical-grade" ethylene which typically has a purity of less than 95%. The impurities typically comprise, in particular, ethane, propane and/or propene.

The reaction or oxidation of ethylene to ethylene oxide is usually carried out at elevated catalyst temperatures. Preference is given to catalyst temperatures in the range of 150 to 350 °C, more preferably 180 to 300 °C, particularly preferably 190 to 280 °C and especially preferably 200 to 280 °C. The present invention therefore also provides a process as described above in which the oxidation is carried out at a catalyst temperature in the range 180 to 300 °C, preferably 200 to 280 °C. Catalyst temperature can be determined by thermocouples located inside the catalyst bed. As used herein, the catalyst temperature or the temperature of the catalyst bed is deemed to be the weight average temperature of the catalyst particles.

The reaction according to the invention (oxidation) is preferably carried out at pressures in the range of 5 to 30 bar. All pressures herein are absolute pressures, unless noted otherwise. The oxidation is more preferably carried out at a pressure in the range of 5 to 25 bar, such as 10 bar to 24 bar and in particular 14 bar to 23 bar. The present invention therefore also provides a process as described above in which the oxidation is carried out at a pressure in the range of 14 bar to 23 bar.

It has been found that the physical characteristics of the catalyst, especially the BET surface area, the pore size distribution, and shape of the catalyst body, have a significant positive impact on the catalyst selectivity. This effect is especially distinguished when the catalyst is operated at very high work rates, i.e., high levels of olefin oxide production.

The process according to the invention is preferably carried out under conditions conducive to obtain a reaction mixture containing at least 2.3 vol.-% of ethylene oxide. In other words, the ethylene oxide outlet concentration (ethylene oxide concentration at the reactor outlet) is preferably at least 2.3 vol.-%. The ethylene oxide outlet concentration is more preferably in the range of 2.5 to 4.0 vol.-%, most preferably in the range of 2.7 to 3.5 vol.-%.

The oxidation is preferably carried out in a continuous process. If the reaction is carried out continuously, the GHSV (gas hourly space velocity) is, depending on the type of reactor chosen, for example on the size/cross-sectional area of the reactor, the shape and size of the catalyst, preferably in the range from 800 to 10,000/h, preferably in the range from 2,000 to 8,000/h, more preferably in the range from 2,500 to 6,000/h, most preferably in the range from 4,500 to 5,500/h, where the values indicated are based on the volume of the catalyst.

According to a further embodiment, the present invention is also directed to a process for preparing ethylene oxide (EO) by gas-phase oxidation of ethylene by means of oxygen as disclosed above, wherein the EO-space-time-yield measured is greater than 180 $kg_{EO}/(m^3_{cat}h)$, preferably to an EO-space-time-yield of greater than 200 $kg_{EO}/(m^3_{cat}h)$, such as greater than 250 $kg_{EO}/(m^3_{cat}h)$, greater than 280 $kg_{EO}/(m^3_{cat}h)$, or greater than 300 $kg_{EO}/(m^3_{cat}h)$. Preferably the EO-space-time-yield measured is less than 500 $kg_{EO}/(m^3_{cat}h)$, more preferably the EO-space-time-yield is less than 350 $kg_{EO}/(m^3_{cat}h)$.

The preparation of ethylene oxide from ethylene and oxygen can advantageously be carried out in a recycle process. After each pass, the newly formed ethylene oxide and the by-products formed in the reaction are removed from the product gas stream. The remaining gas stream is supplemented with the required amounts of ethylene, oxygen and reaction moderators and reintroduced into the reactor. The separation of the ethylene oxide from the product gas stream and its work-up can be carried out by customary methods of the prior art (cf. Ullmann's Encyclopedia of Industrial Chemistry, 5th edition, vol. A-10, pp. 117-135, 123-125, VCH-Verlagsgesellschaft, Weinheim 1987).

The invention will be described in more detail by the accompanying drawings and the subsequent examples.

Fig. 1a, 1b, 1c and 1d show schematic cross-sections of shaped catalyst bodies of the invention.

Fig. 2a, 2b, 2c, 2d, 2e and 2f show schematic cross-sections of shaped catalyst bodies according to Fig. 1a, 1b, 1c and 1d.

Fig. 3a, 3b, and 3c show schematic side-views of shaped catalyst bodies according to Fig. 1a, 1b, and 1d.

Fig. 4 shows the pore size distribution of a preferred refractory support.

Fig. 5 shows the pore size distribution of a preferred shaped catalyst body according to the invention.

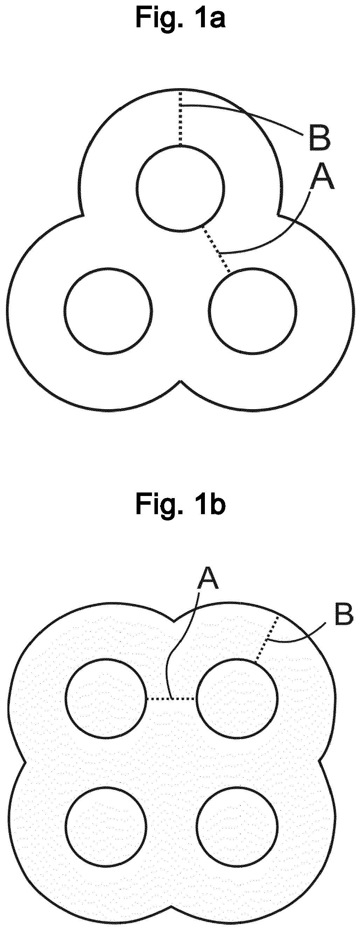

With regard to Fig. 1a, the shaped catalyst body has three void spaces running in the cylinder periphery to form a trilobed structure, wherein the cross-section of the trilobed structure has the shape of an equilateral triangle with an elliptical segment attached to each side. The shaped catalyst body has three passageways with a circular cross-section, arranged equidistantly to each other. The centers of the three passageways form an equilateral triangle, and each passageway is arranged symmetrically within a lobe. The shaped catalyst body has a 3-fold rotational symmetry.

With regard to Fig. 1b, the shaped catalyst body has four void spaces running in the cylinder periphery to form a tetralobed structure, wherein the cross-section of the tetralobed structure has the shape of a square with an elliptical segment attached to each side. The shaped catalyst body has four passageways with a circular cross-section, arranged equidistantly to each other. The centers of the four passageways form a square, and each passageway is arranged symmetrically within a lobe. The shaped catalyst body has a 4-fold rotational symmetry.

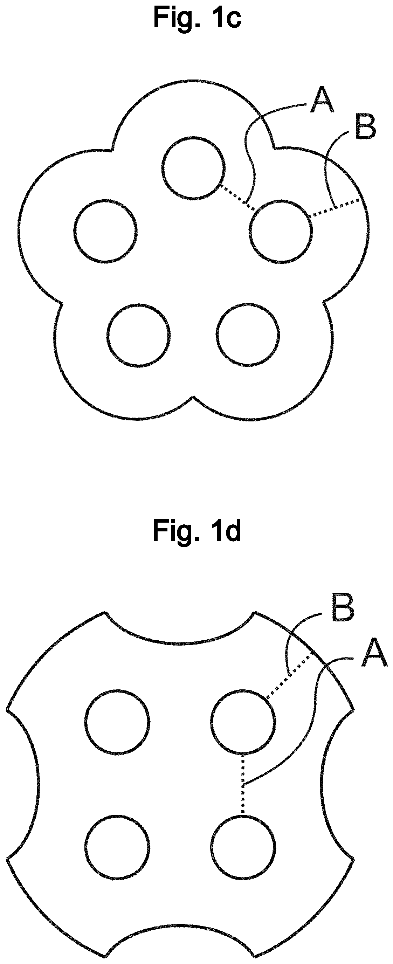

With regard to Fig. 1c, the shaped catalyst body has five void spaces running in the cylinder periphery to form a pentalobed structure, wherein the cross-section of the pentalobed structure has the shape of an equilateral, equiangular pentagon with an elliptical segment attached to each side. The shaped catalyst body has five passageways with a circular cross-section, arranged equidistantly to each other. The centers of the five passageways form an equilateral, equiangular pentagon, and each passageway is arranged symmetrically within a lobe. The shaped catalyst body has a 5-fold rotational symmetry.

With regard to Fig. 1d, the shaped catalyst body has four void spaces running in the cylinder periphery to form a tetralobed structure, wherein the cross-section of the tetralobed structure has the shape of a circle with four indentations, each indentation having the shape of an elliptical segment. The shaped catalyst body has four passageways with a circular cross-section, arranged equidistantly to each other. The centers of the four passageways form a square, and each passageway is arranged symmetrically within a lobe. The shaped catalyst body has a 4-fold rotational symmetry.

For illustrative purposes, Fig. 1a, 1b, 1c and 1d each show the shortest distance A between two neighboring passageways, as well as the shortest distance B between a passageway and the circumferential surface.

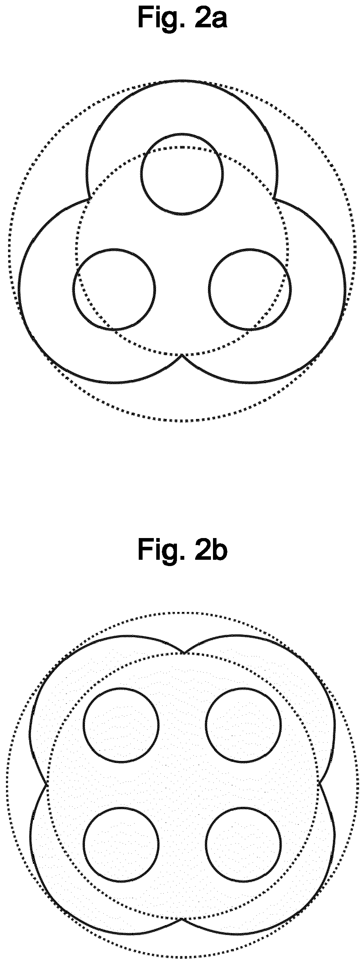

Fig. 2a shows a schematic cross-section of a shaped catalyst body according to Fig. 1a. The circumscribed circle and the inscribed circle are indicated by dashed lines.

Fig. 2b shows a schematic cross-section of a shaped catalyst body according to Fig. 1b. The circumscribed circle and the inscribed circle are indicated by dashed lines.

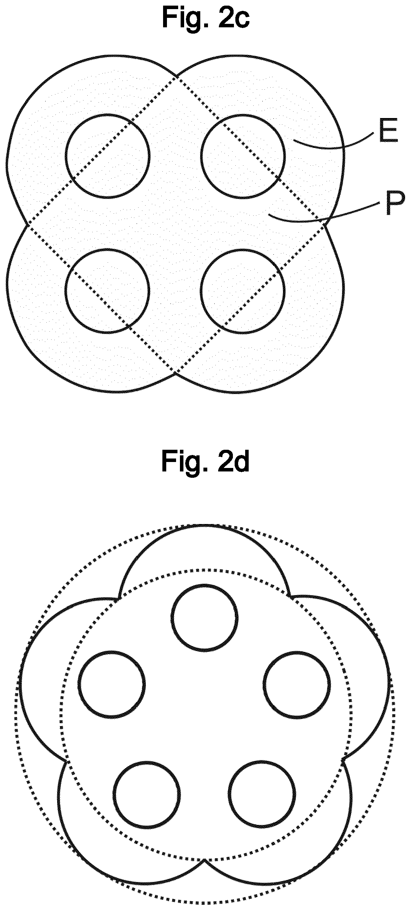

Fig. 2c shows a schematic cross-section of a shaped catalyst body according to Fig. 1b. The cross-section is derived from a circular-cylinder structure with a plurality of void spaces. The cross-section has the shape of a substantially equilateral, equiangular polygon with elliptical segments attached to each side. In Fig. 2c, the sides of the substantially equilateral, equiangular polygon P are indicated by dashed lines. The elliptical segments E traverse the side they are attached to and meet at the corners of the polygon P.

Fig. 2d shows a schematic cross-section of a shaped catalyst body according to Fig. 1c. The circumscribed circle and the inscribed circle are indicated by dashed lines.

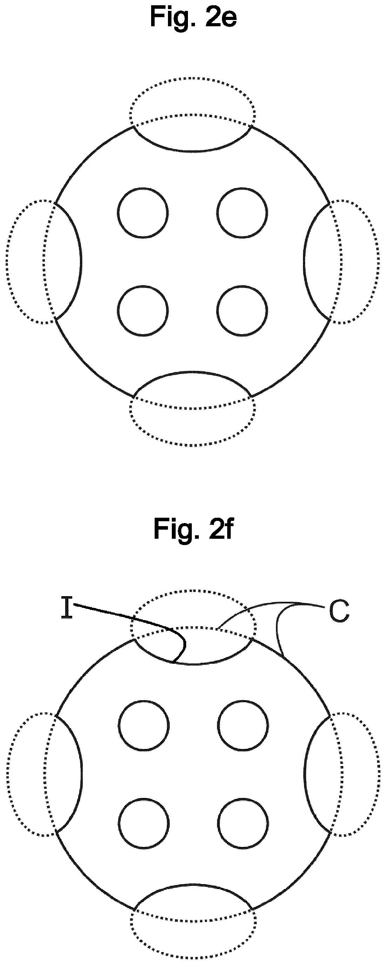

Fig. 2e shows a schematic cross-section of a shaped catalyst body according to Fig. 1d. The circumscribed circle and the inscribed circle are indicated by dashed lines.

Fig. 2f shows a schematic cross-section of a shaped catalyst body according to Fig. 1d. The cross-section is derived from a circular-cylinder structure with a plurality of void spaces. The cross-section has the shape of a circle with four indentations, each indentation having the shape of an elliptical segment. In Fig. 2f, circle C and indentations I are indicated. Where circle C is indented, the underlying shape of the circle is indicated by dashed lines. Further, the elliptical shape underlying the shape of the indentations is indicated by dashed lines.

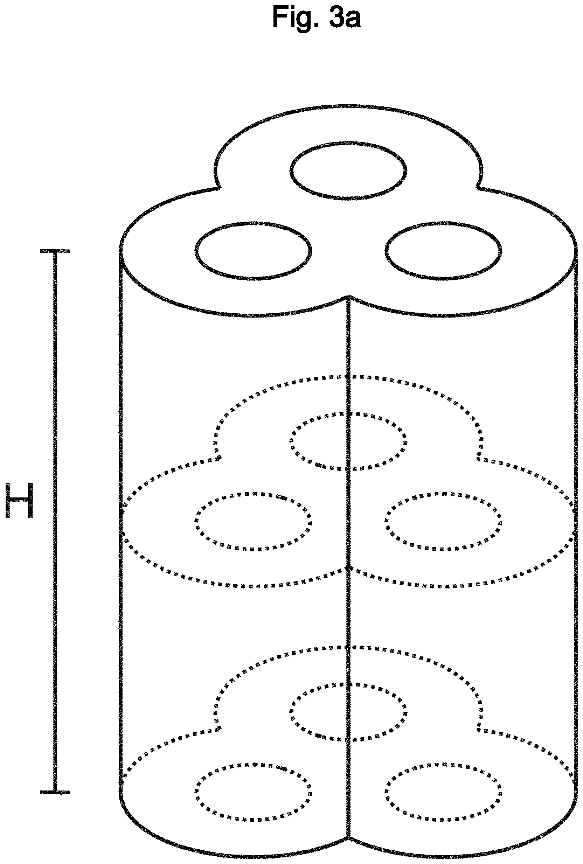

Fig. 3a shows a schematic side-view of a shaped catalyst body according to Fig. 1a, wherein cross-sections are shown as dashed lines. The height h of the shaped catalyst body is indicated.

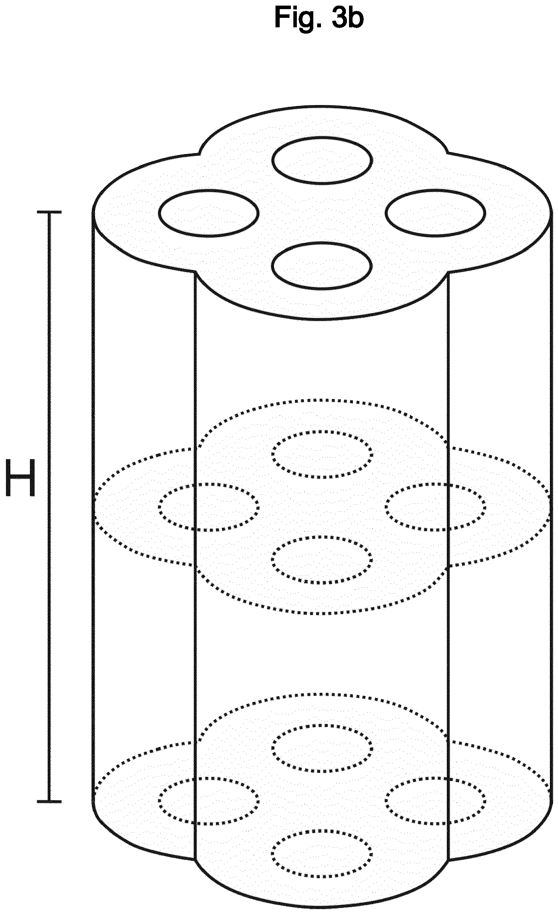

Fig. 3b shows a schematic side-view of a shaped catalyst body according to Fig. 1b, wherein cross-sections are shown as dashed lines. The height h of the shaped catalyst body is indicated.

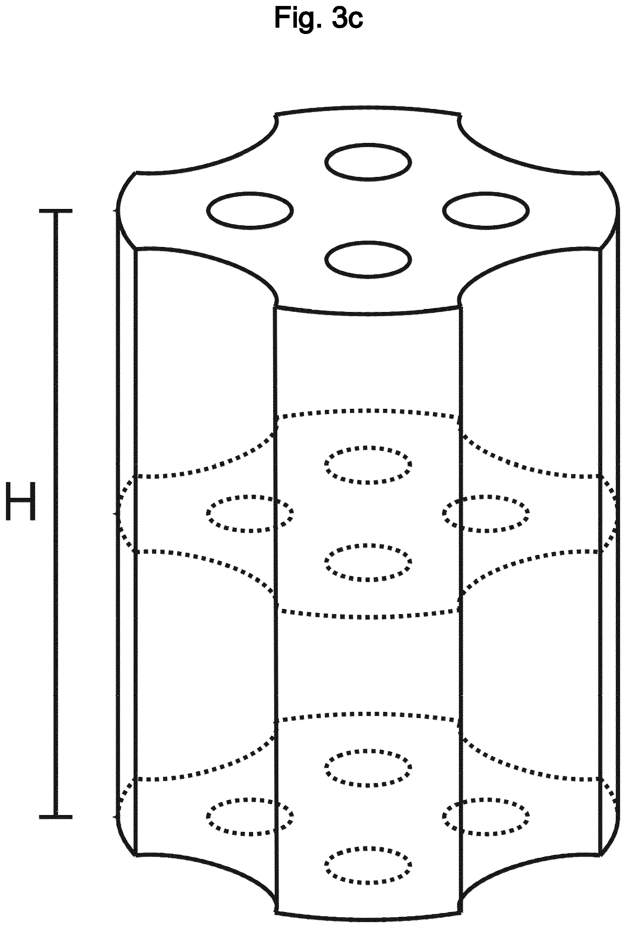

Fig. 3c shows a schematic side-view of a shaped catalyst body according to Fig. 1d, wherein cross-sections are shown as dashed lines. The height h of the shaped catalyst body is indicated.

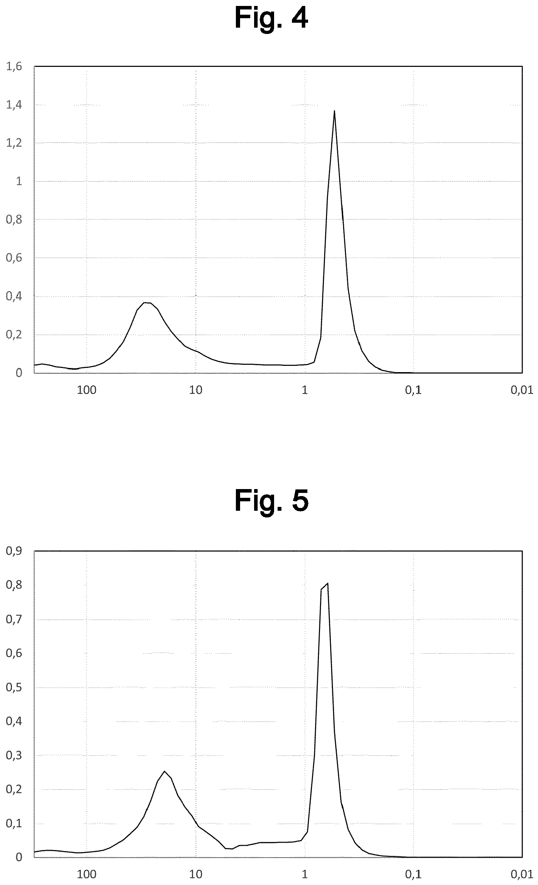

Fig. 4 shows the pore size distribution of the refractory support B described below, as determined by mercury porosimetry. On the x-axis, the pore size diameter [$\mu$m] is plotted. On the y-axis, the log differential intrusion [mL/g] is plotted.

Fig. 5 shows the pore size distribution of the catalyst 1-1 described below, as determined by mercury porosimetry. On the x-axis, the pore size diameter [$\mu$m] is plotted. On the y-axis, the log differential intrusion [mL/g] is plotted in. Fig. 5 shows a multimodal pore size distribution with a first pore size distribution maximum in the range of 0.1 to 3.0 $\mu$m and a second pore size distribution maximum in the range of 8.0 to 100 $\mu$m, wherein at least 40% of the total Hg pore volume of the shaped catalyst body stems from pores with a diameter in the range of 0.1 to 3.0 $\mu$m.

Examples

Methods and Materials

Method 1 - Experimental Measurement of the Pressure Drop

The pressure drop coefficient $\xi$ is proportional to the pressure drop and is defined as

$$\xi = \frac{\Delta p}{H} \cdot d_k \cdot \frac{2}{\rho \cdot w^2}$$

with the pressure drop $\Delta p$ in Pascal, the height of the packing H in meters, the constant reference length $d_K$ of 0.01 meters, the average gas density $\rho$ in kg/m$^3$ and the average superficial gas velocity w in m/s.

The pressure drop coefficient $\xi$ can be described using the following equalization function:

$$\xi = a + \frac{b}{Re_{dk10}}$$

where the Reynolds number $Re_{dk10}$ is defined as

$$Re_{dk10} = \frac{w \cdot d_k \cdot \rho}{\eta}$$

with the dynamic viscosity of the gas $\eta$ in Pascal·seconds. The parameters a and b can be obtained by linear regression from experimental values, as measured in a tube with an internal diameter typical of a technical reactor (e.g., 26 or 39 mm). A suitable Reynolds number in an ethylene oxide process is between 5,000 and 6,000, for example 5,500.

Method 2 - Mercury Porosimetry

Mercury porosimetry was performed using a Micrometrics AutoPore IV 9500 mercury porosimeter (140 degrees contact angle, 485 dynes/cm Hg surface tension, 60000 psia max head pressure). The Hg porosity is determined in accordance with DIN 66133.

Method 3 - BET Surface Area

The BET surface area was determined in accordance with DIN ISO 9277.

Method 4 - Water Absorption

Water absorption refers to vacuum cold water uptake. Vacuum cold water uptake is determined by placing about 100 g of support ("initial support weight") in a rotating flask, covering the support with deionized water, and rotating the rotary evaporator for 5 min at about 30 rpm. Subsequently, a vacuum of 80 mbar is applied for 3 min, the water and the support are transferred into a glass funnel, and the support is kept in the funnel for about 5 min with occasional shaking in order to ensure that adhering water runs down the funnel.
The support is weighed ("final support weight"). The water absorption is calculated by subtracting the initial support weight from the final support weight and then dividing this difference by the initial support weight.

Method 5 - Side Crush Strength

The side crush strength was determined using an apparatus of the "Z 2.5 / T 919" type supplied by Zwick Roll (Ulm), stamp size: 12.7 mm $\times$ 12.7 mm. Based on measurements of 25 randomly selected shaped bodies, average values were calculated.
In the measurements of trilobes, the force is exerted along an axis running through a first passageway and the center of the circumscribed circle. The measurements of tetralobes were performed along two directions - along the side and along the diagonal. In the measurement along the diagonal, the force is exerted along an axis running through a first passageway and a second passageway opposite to the first passageway. In the measurement along the side, the force is exerted along two axes each running through two adjacent passageways.

Method 6 - Packing tube density

Packing tube density was determined by filling catalyst shaped bodies in a glass tube with an inner diameter of 26 mm or 39 mm to a marker marking an inner tube volume of 1L.

Method 7 - Simulation of Geometric Dimensions, Geometric Surface and Geometric Volume

A CAD (Computer Aided Design) model of a single shaped catalyst body is created with any CAD program to calculate the geometric dimensions, geometric surface and geometric volume.

Refractory Supports

The refractory supports were alumina supports and comprised Si, Ca, Mg, Na, K and Fe as chemical impurities. The properties of the refractory supports are indicated in Table 1 below. The supports were obtained from EXACER s.r.l. (Via Puglia 2 /4, 41049 Sassuolo (MO), Italy), under the following lot numbers:

| A | T 405 |
| B | T 915 |
| C | F 916 |

The supports A to C have $Si_{Al2O3}$ = 600 ppm, $Ca_{Al2O3}$ = 400 ppm, $Mg_{Al2O3}$ = 200 ppm, $Na_{Al2O3}$ = 100 ppm, $K_{Al2O3}$ = 200 ppm, $Fe_{Al2O3}$ = 100 ppm. Supports A to C have a bimodal pore size distribution with smaller pores with the first log differential pore volume distribution peak in the range of 0.4 to 0.6 $\mu$m and the second log differential pore volume distribution peak in the range of 12 to 21 $\mu$m.

Table 1

| | A[1] | B | C |
|---|---|---|---|
| Shape | ring | trilobe | tetralobe |
| Passageways | 1 | 3 | 4 |
| diameter of circumscribed circle $d_{cc}$ [mm] | 7.5 | 7.1 | 7.4 |
| diameter of inscribed circle $d_{ic}$ [mm] | 7.5 | 4.4 | 5.7 |
| quotient $d_{ic}$ / $d_{cc}$ | 1 | 0.62 | 0.77 |
| height h [mm] | 8.3 | 7.0 | 6.8 |
| quotient $d_{cc}$ / h | 0.90 | 1.01 | 1.09 |
| diameter of passageways [mm] | 2.15 | 1.43 | 1.3 |
| shortest distance A [mm] | - | 1.25 | 1.05 |
| shortest distance B [mm] | 2.7 | 1.29 | 1.37 |
| ratio of shortest distance A to shortest distance B | - | 0.97 | 0.77 |
| ratio of total cross-sectional area of passageways to cross-sectional area of shaped catalyst body | 0.08 | 0.17 | n.d.[6] |
| geometric surface area $SA_{geo}$ [mm$^2$] | 333 | 291 | n.d. |
| geometric volume $V_{geo}$ [mm$^3$] | 336 | 173 | n.d. |
| quotient $SA_{geo}$ / $V_{geo}$ [mm$^{-1}$] | 0.99 | 1.69 | n.d. |
| BET surface area [m$^2$/g] | 2.0 | 2.03 | 2.07 |
| water uptake [mL/g] | 0.55 | 0.53 | 0.57 |
| total Hg pore volume [mL/g] | 0.55 | 0.54 | 0.50 |
| 1st log diff. peak [$\mu$m][2] | 0.6 | 0.5 | 0.5 |
| 2nd log diff. peak [$\mu$m][2] | 12 | 30 | 22 |
| Passageways | 1 | 3 | 4 |
| Percentage of pores with a diameter of 0.1 to 3 $\mu$m [%][3] | n.d. | 55 | 55 |
| carrier density [g/L] [4] | 590 | 543 | 538 |
| side crush strength [N] along diagonal | 58 | - | 49 |
| side crush strength [N] along one lobe | - | 47 | - |
| side crush strength [N] along side | - | - | 86 |
| a | 26.1 | 30.4 | n.d. |
| b | 2555 | 4355 | n.d. |
| Relative pressure drop coefficient at Re = 5,500 [%][5] | 100 | 120 | n.d. |

[1] Reference Example
[2] log diff. peak = log differential pore volume distribution peak
[3] based on the total Hg pore volume
[4] measured in a tube with a diameter of 26 mm
[5] measured in a tube with a diameter of 39 mm; values are relative to support A
[6] n.d. = not determined

Example 1 - Preparing Shaped Catalyst Bodies

Shaped catalyst bodies according to Tables 2, 3 below were prepared by impregnating the refractory supports disclosed in Table 1 with a silver impregnation solution. The supports were obtained from EXACER s.r.l. (Via Puglia 2 /4, 41049 Sassuolo (MO), Italy), under the lot numbers shown above.

Table 2: Catalyst composition (Ag-contents are reported in percent by weight of total catalyst, dopant values are reported in parts per million by weight of total catalyst)

| Example | Support | $Ag_{CAT}$ [wt-%] | $Li_{CAT}$ [ppm] | $S_{CAT}$ [ppm] | $W_{CAT}$ [ppm] | $Cs_{CAT}$ [ppm] | $Re_{CAT}$ [ppm] | $K_{ADD}$ [ppm] | $K_{CAT}$ [ppm] |
|---|---|---|---|---|---|---|---|---|---|
| 1-1 | B | 28.8 | 470 | 35 | 540 | 950 | 1150 | 98 | 229 |

Table 3: Physical Properties of Catalysts

| Catalyst | 1-1 |
|---|---|
| Support | B |
| Shape | trilobe |
| Number of passageways in the carrier | 3 |
| **Physical Properties** | |
| BET surface area [$m^2$/g] | 2.5 |
| Total Hg pore volume [ml/g] | 0.34 |
| 1$^{st}$ log diff peak [$\mu$m] | 0.6 |
| 2$^{nd}$ log diff peak [$\mu$m] | 19 |
| Percentage of pores 0.1 to 3 $\mu$m in relation to the total Hg pore volume [%] | 56 |
| Percentage of pores 8 to 100 $\mu$m in relation to the total Hg pore volume [%] | 36 |
| Catalyst density in 26 mm tube [g/L] | 833 |
| side crush strength [N] along one lobe | 75 |

1.1 Production of the Silver Complex Solution

783 kg of an aqueous ethylenediamine solution with an ethylenediamine content of 59 wt% were pumped into a stirring reactor 1. Subsequently, the ethylenediamine solution was diluted under stirring with 94 kg of deionized water. Next, 26.6 kg of 0.95 wt% KOH solution were added to form an aqueous KOH / ethylenediamine solution. The solution was cooled to a temperature of below 20 °C. Then 300 kg of oxalic acid dihydrate (purity $\geq$ 99.6 %) were added into the stirring reactor 1 stepwise over a period of about 180 minutes under stirring and cooling to control the reaction temperature in the range of 20 to 25 °C. Once the addition of oxalic acid dihydrate was completed and the temperature profile from the addition of the last portion of oxalic acid dihydrate passed a maximum, cooling was terminated and the reaction mixture was stirred further for the next 60 min to form an aqueous oxalic acid / ethylenediamine solution.

Next, 1113 kg of the aqueous oxalic acid / ethylenediamine solution were transferred from the stirring reactor 1 to a stirring reactor 2. The reaction medium was cooled to a temperature below 20 °C. Then, 500 kg silver oxide powder (from Ames Goldsmith, chemical composition shown below), was added over a period of 225 min under stirring and cooling to control the reaction temperature in the range of 20 to 25 °C. Once the addition of silver oxide was completed, and the temperature profile from the addition of the last portion of silver oxide passed a maximum, cooling was terminated and the reaction mixture was heated under stirring in a temperature range of about 25 - 30°C for the next 3 hours to form an aqueous Ag complex suspension.

The silver oxide used is commercially available from Ames Goldsmith. Its chemical composition is described below:

| Silver Content as $Ag_2O$ | $\geq$ 99.90% |
|---|---|
| moisture content | $\leq$ 0.20% |
| chlorides | $\leq$ 15 ppm |

(continued)

| | |
|---|---|
| nitrates | ≤ 100 ppm |
| carbonates | ≤ 0.25% |
| sulfates | ≤ 20 ppm |
| copper | ≤ 20 ppm |
| iron | ≤ 20 ppm |
| lead | ≤ 20 ppm |
| nickel | ≤ 20 ppm |
| sodium | ≤ 50 ppm |
| other trace metals | ≤ 20 ppm |

Subsequently, the silver impregnation solution was obtained by passing the reaction mixture through a filtration unit SUPRAdisc® II KS 100 Depth Filter Modules available from Pall Corporation, Port Washington, USA, to remove a minor amount of undissolved solid. The resulting silver impregnation solution had a density of 1.530 g/mL and a silver content of 29.3 wt-%.

1.2 Preparation of Shaped Catalyst Bodies

Step 1.2.1. Preparation of Ag-containing intermediate

324.1 g of support B listed in Table 1 was placed into a 2L glass flask. The flask was attached to a rotary evaporator which was set under vacuum pressure of 80 mbar. The rotary evaporator system was set in rotation of 30 rpm. 257.8 of silver complex solution prepared according to step 1.1 was added onto support B over 15 minutes under vacuum of 80 mbar. After addition of the silver complex solution, the rotary evaporator system was continued to rotate under vacuum for another 15 minutes. The impregnated support was then left in the apparatus at room temperature and atmospheric pressure for 1 hour and mixed gently every 15 minutes. The impregnated support was calcined for 12 minutes at 290°C under 23 m³/h flowing nitrogen in a calcination oven to yield Ag-containing intermediate product.

Step 1.2.2. Preparation of final catalyst 1-1 with properties listed in Tables 2, 3.

398.7 g of Ag-containing intermediate product prepared according to step 2.2.1 were placed into a 2L glass flask. The flask was attached to a rotary evaporator which was set under vacuum pressure of 80 mbar. The rotary evaporator system was set in rotation of 30 rpm. 192.07 g of the silver complex solution prepared according to step 1.1 was mixed with 7.53 g of promoter solution I, 8.22 g of promoter solution II, and 14.19 g of promoter solution III. Promoter solution I was made from dissolving lithium nitrate (FMC, 99.3%) and ammonium sulfate (Merck, 99.4%) in DI water to achieve Li content of 2.85 wt% and S content of 0.21 wt%. Promoter solution II was made from dissolving tungstic acid (HC Starck, 99.99%) in DI water and cesium hydroxide in water (HC Starck, 50.42%) to achieve target Cs content of 5.28 wt% and W content of 3.0 wt%. Promoter solution III was made from dissolving ammonium perrhenate (Engelhard, 99.4%) in DI water to achieve Re content of 3.7 wt%. The combined impregnation solution containing silver complex solution, promoter solutions I, II, and III was stirred for 5 minutes. The combined impregnation solution was added onto the silver-containing intermediate product 1.2.1 over 15 minutes under vacuum of 80 mbar. After addition of the combined impregnation solution, the rotary evaporator system was continued to rotate under vacuum for another 15 minutes. The impregnated support was then left in the apparatus at room temperature and atmospheric pressure for 1 hour and mixed gently every 15 minutes. The impregnated material was calcined for 10 minutes at 290°C under 23 m³/h flowing nitrogen in a calcination oven to yield the final catalyst 1-2.

**Claims**

1. A shaped catalyst body for producing ethylene oxide by gas-phase oxidation of ethylene, comprising silver deposited on a porous refractory support, the shaped catalyst body having a first face side surface, a second face side surface and a circumferential surface, **characterized by**

- a content of at least 20 wt.-% of silver, relative to the total weight of the shaped catalyst body;
- a cylinder structure with n void spaces running in the cylinder periphery along the cylinder height to form an n-lobed structure, wherein n is 2, 3, 4, 5 or 6;
- n passageways extending from the first face side surface to the second face side surface, each passageway being assigned to one lobe, wherein neighboring passageways are arranged essentially equidistantly to each other;
- an n-fold rotational symmetry;
- a shortest distance A between two neighboring passageways in the range of 1.0 to 2.0 mm;
- a shortest distance B between each passageway and the circumferential surface in the range of 1.1 to 2.0 mm; and
- a BET surface area in the range of 1.6 to 3.0 $m^2/g$.

2. The shaped catalyst body according to claim 1, wherein the cross-section of the n-lobed structure has the shape of a circle with n indentations, each indentation having the shape of an elliptical segment.

3. The shaped catalyst body according to claim 1, wherein the cross-section of the n-lobed structure has the shape of a substantially equilateral, equiangular polygon having n sides, with elliptical segments attached to each side.

4. The shaped catalyst body according to any one of the preceding claims, wherein the passageways have an elliptical cross-section.

5. The shaped catalyst body according to any one of the preceding claims, wherein the ratio of the shortest distance A to the shortest distance B is in the range of 0.6 to 1.3.

6. The shaped catalyst body according to any one of the preceding claims, wherein a quotient of the geometric surface of the shaped catalyst body $SA_{geo}$ over the geometric volume of the shaped catalyst body $V_{geo}$ is at least 1.1 $mm^{-1}$ and at most 10 $mm^{-1}$.

7. The shaped catalyst body according to any one of the preceding claims, wherein the cross-section of the n-lobed structure has a quotient of the diameter of the inscribed circle $d_{ic}$ over the diameter of the circumscribed circle $d_{cc}$ of at most 0.9, preferably in the range of 0.55 to 0.85.

8. The shaped catalyst body according to any one of the preceding claims, wherein the cross-sectional area of each of the passageways is independently in the range of 0.5 to 13.0 $mm^2$, preferably in the range of 1.0 to 3.2 $mm^2$.

9. The shaped catalyst body according to any one of the preceding claims, wherein the ratio of the total cross-sectional area of the passageways to the cross-sectional area of the shaped catalyst body is in the range of 0.1 to 0.4.

10. The shaped catalyst body to any one of the preceding claims, having a height h in the range of 6 to 12 mm, preferably in the range of 7 to 11 mm.

11. The shaped catalyst body to any one of the preceding claims, wherein the quotient of the diameter of the circumscribed circle $d_{cc}$ over the height h is in the range of 0.25 to 2.0, preferably in the range of 0.5 to 1.5.

12. The shaped catalyst body to any one of the preceding claims, wherein the shaped catalyst body has a side crush strength of at least 50 N.

13. The shaped catalyst body according to any one of the preceding claims, wherein the shaped catalyst body comprises 400 to 2000 ppm by weight of rhenium, relative to the total weight of the shaped catalyst body.

14. The shaped catalyst body according to any one of the preceding claims, wherein the shaped catalyst body comprises 260 to 1750 ppm by weight of cesium, relative to the total weight of the shaped catalyst body.

15. The shaped catalyst body according to any one of the preceding claims, wherein the refractory support is an aluminium oxide support.

16. The shaped catalyst body according to any one of the preceding claims, wherein the shaped catalyst body has a total Hg pore volume of 0.2 mL/g to 0.6 mL/g, as determined by Hg intrusion measurements.

17. The shaped catalyst body according to claim 16, wherein the catalyst has a multimodal pore size distribution with a first pore size distribution maximum in the range of 0.1 to 3.0 $\mu$m and a second pore size distribution maximum in the range of 8.0 to 100 $\mu$m, wherein at least 40% of the total Hg pore volume of the shaped catalyst body stems from pores with a diameter in the range of 0.1 to 3.0 $\mu$m.

18. A reactor tube packed with a plurality of shaped catalyst bodies according to any one of the preceding claims, wherein the ratio of the diameter of the circumscribed circle $d_{cc}$ of the shaped catalyst bodies to the inner diameter of the reactor tube is in the range of 0.15 to 0.35, preferably 0.2 to 0.3.

19. A process for producing ethylene oxide by gas-phase oxidation of ethylene, comprising reacting ethylene and oxygen in the presence of a shaped catalyst body as defined in any one of claims 1 to 17.

20. A process for preparing a shaped catalyst body as defined in any one of claims 1 to 17, comprising

i) impregnating a refractory support having a BET surface area in the range of 1.4 to 2.5 m$^2$/g with a silver impregnation solution; and
ii) subjecting the impregnated refractory support to a calcination process; wherein steps i) and ii) are optionally repeated.

# Fig. 1a

# Fig. 1b

# Fig. 1c

# Fig. 1d

# Fig. 2a

# Fig. 2b

# Fig. 2c

E

P

# Fig. 2d

# Fig. 2e

# Fig. 2f

## Fig. 3a

# Fig. 3b

# Fig. 3c

# Fig. 4

# Fig. 5

Europäisches
Patentamt
European
Patent Office
Office européen
des brevets

## EUROPEAN SEARCH REPORT

Application Number

EP 18 20 8856

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2009/114411 A2 (SD LIZENZVERWERTUNGSGESELL MBH [DE]; PAK SERGUEI [US] ET AL.) 17 September 2009 (2009-09-17) * abstract * * paragraph [0001] * * paragraph [0014] - paragraph [0028] * * paragraph [0033] * * paragraph [0044] * * paragraph [0055]; claims; examples * ----- | 1-20 | INV. B01J37/02 B01J19/30 B01J23/50 B01J23/66 B01J23/68 B01J35/02 B01J35/10 C07D301/10 |
| Y | WO 2018/102377 A1 (SHELL OIL CO [US]; SHELL INT RESEARCH [NL]) 7 June 2018 (2018-06-07) * abstract * * page 1, paragraph 18-24 * * page 2, line 20 - page 3, line 12 * * page 15, line 15 - page 16, line 31 * * page 18, lines 9-24 * * page 19, line 6 - page 21, line 25 * * page 26, lines 13-24 * ----- | 1-20 | |
| Y | WO 2018/029189 A1 (BASF SE [DE]) 15 February 2018 (2018-02-15) * abstract * * page 1, lines 5-10 * * page 3, line 30 - page 4, line 27 * * page 6, line 40 - page 8, line 3 * * page 11, line 6 - page 12, line 6 * * claims; examples * ----- -/-- | 1-20 | TECHNICAL FIELDS SEARCHED (IPC) B01J C07D |

The present search report has been drawn up for all claims

| Place of search | Date of completion of the search | Examiner |
|---|---|---|
| Munich | 9 August 2019 | Nazario, Luis |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 2

Europäisches
Patentamt

European
Patent Office

Office européen
des brevets

## EUROPEAN SEARCH REPORT

Application Number

EP 18 20 8856

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2004/014549 A1 (COMBS GLENN A [US]) 19 February 2004 (2004-02-19) * abstract * * page 1, lines 5-7 * * page 7, lines 3-9 * * page 17, line 29 - page 18, line 4 * * page 24, line 23 - page 25, line 29; claims; figures 1, 2, 19, 20, 25 * ----- | 1-20 | |
| A | US 2012/264954 A1 (ROSENDAHL TOBIAS [DE] ET AL) 18 October 2012 (2012-10-18) * abstract * * paragraphs [0009], [0012], [0022], [0029], [0038], [0045] - [0047], [0053]; claims; figures 4, 8 * ----- | 1-20 | |
| A | EP 0 678 331 A1 (MONTECATINI TECNOLOGIE SRL [IT]) 25 October 1995 (1995-10-25) * abstract * * page 2, line 40 - page 3, line 30; claims; figure 1; examples; tables * ----- | 1-20 | |

TECHNICAL FIELDS
SEARCHED     (IPC)

The present search report has been drawn up for all claims

| Place of search | Date of completion of the search | Examiner |
|---|---|---|
| Munich | 9 August 2019 | Nazario, Luis |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 20 8856

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2019

| Patent document cited in search report | | Publication date | Patent family member(s) | | Publication date |
|---|---|---|---|---|---|
| WO 2009114411 | A2 | 17-09-2009 | BR | PI0909058 A2 | 24-11-2015 |
| | | | CA | 2717652 A1 | 17-09-2009 |
| | | | CN | 101965224 A | 02-02-2011 |
| | | | EP | 2252398 A2 | 24-11-2010 |
| | | | JP | 2011514845 A | 12-05-2011 |
| | | | KR | 20110014139 A | 10-02-2011 |
| | | | RU | 2010141552 A | 20-04-2012 |
| | | | TW | 200946227 A | 16-11-2009 |
| | | | US | 2009227820 A1 | 10-09-2009 |
| | | | WO | 2009114411 A2 | 17-09-2009 |
| WO 2018102377 | A1 | 07-06-2018 | CA | 3043504 A1 | 07-06-2018 |
| | | | CN | 110035998 A | 19-07-2019 |
| | | | KR | 20190088049 A | 25-07-2019 |
| | | | TW | 201822883 A | 01-07-2018 |
| | | | WO | 2018102377 A1 | 07-06-2018 |
| WO 2018029189 | A1 | 15-02-2018 | CN | 109562358 A | 02-04-2019 |
| | | | EP | 3496851 A1 | 19-06-2019 |
| | | | WO | 2018029189 A1 | 15-02-2018 |
| WO 2004014549 | A1 | 19-02-2004 | AU | 2003264040 A1 | 25-02-2004 |
| | | | US | 2004043900 A1 | 04-03-2004 |
| | | | WO | 2004014549 A1 | 19-02-2004 |
| US 2012264954 | A1 | 18-10-2012 | NONE | | |
| EP 0678331 | A1 | 25-10-1995 | AU | 689537 B2 | 02-04-1998 |
| | | | CA | 2146184 A1 | 06-10-1995 |
| | | | DE | 69508637 D1 | 06-05-1999 |
| | | | DE | 69508637 T2 | 30-09-1999 |
| | | | DK | 0678331 T3 | 11-10-1999 |
| | | | EP | 0678331 A1 | 25-10-1995 |
| | | | ES | 2131231 T3 | 16-07-1999 |
| | | | IT | 1274033 B | 14-07-1997 |
| | | | JP | 3781453 B2 | 31-05-2006 |
| | | | JP | H0838903 A | 13-02-1996 |
| | | | KR | 950031222 A | 18-12-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012091898 A2
- WO 2004101144 A1
- US 20150119590 A1
- US 4837194 A
- WO 2009114411 A2
- CN 102688784 A
- DE 102005019596 A
- EP 0902726 A
- EP 1478458 A
- EP 1675678 A
- EP 3254756 A
- WO 2004101144 A
- WO 2007021472 A
- WO 2008054654 A
- WO 2009029414 A
- WO 2010008920 A
- WO 2011153390 A1
- WO 2012143557 A1
- WO 2012143559 A1
- WO 2013077839 A1
- US 20180021755 A
- WO 2013066557 A
- WO 2018044992 A
- US 5504052 A
- US 5646087 A
- US 7553795 B
- US 8378129 B
- US 8546297 B
- US 20140187417 A
- EP 1893331 A

- WO 2012140614 A
- EP 0902726 B1
- WO 2011153390 A2
- EP 0716884 A2
- EP 1115486 A1
- EP 1613428 A1
- US 4731350 A
- WO 2004094055 A2
- WO 2009029419 A1
- WO 2015095508 A1
- US 4356312 A
- US 5187140 A
- US 4908343 A
- US 5504053 A
- WO 2014105770 A1
- DE 3414717 A
- EP 0082609 A
- EP 0339748 A
- US 4921681 A
- WO 2006102189 A
- DE 2521906 A
- EP 0014457 A
- DE 2300512 A
- EP 0172565 A
- DE 2454972 A
- EP 0357293 A
- EP 0266015 A
- EP 0085237 A
- US 6452027 B

### Non-patent literature cited in the description

- Ullmann's Encyclopedia of Industrial Chemistry. VCH-Verlagsgesellschaft, 1987, vol. A-10, 117-135, 123-125